# EUROPÄISCHE PATENTANMELDUNG

(11) **EP 1 555 010 A1**
(43) Veröffentlichungstag der Anmeldung: **20.07.2005**
(21) Anmeldenummer: 04105417.2
(22) Anmeldetag: 29.10.2004
(51) Int. Cl.: A61K 7/075, A61K 7/50

(54) **Kosmetische Reinigungszubereitung mit verbesserten Pflegeeigenschaften**

(30) Priorität: 18.12.2003 DE 20319655 U
(71) Anmelder: Beiersdorf AG, 20245 Hamburg (DE)
(72) Erfinder: Ruppert, Stephan, 20259, Hamurg (DE); Spiegel, Anja, 22085, Hamburg (DE)

(57) **Zusammenfassung**

Kosmetische und/oder dermatologische Körper- und Haarreinigungszubereitung enthaltend
a) Di-n-Octylether oder Di-n-Octylcarbonat
b) mindestens zwei kationische Polymere, wobei es sich bei einem der Polymere um
   b1) ein quarternisiertes Polymer auf Basis von Cellulose, bevorzugt auf Basis von Hydroxypro-pylcellulose und besonders bevorzugt um Polyquaternium-10
   b2) ein quarternisiertes Guar Derivat, bevorzugt um Guar-Hydroxypropyltrimethylammoniumchlorid
handelt.

## Beschreibung

Die vorliegende Erfindung betrifft eine Körperreinigungszubereitung mit verbesserten Pflegeeigenschaften.

Eine Wachsdispersion im Sinne der vorliegenden Schrift ist eine feinteilige Dispersion von festen, partikulären Wachsbestandteilen in einer kontinuierlichen wässrigen Phase, die gegebenenfalls weitere stabilisierende Bestandteile enthält.

Neben der Reinigung ist bei tensidhaltigen Reinigungszubereitungen die Vermittlung von Pflegeleistung ein entscheidender Aspekt. Eine gute Pflegeleistung drückt sich aus in einer Reduzierung der Kämmkraft, einem angenehm seidigen Haargriff und gutem Hautgefühl. Hierzu werden den Shampoos oder Duschbädern Zusatzstoffe zugesetzt, die die Sensorik günstig beeinflussen. Bekannt ist, dass sich der Haargriff durch Zusatz von Dicaprylylether oder Dicaprylylcarbonat günstig beeinflussen lässt und dass dieser Effekt durch den Zusatz von Wachsdispersionen noch gesteigert werden kann. Nachteilig ist jedoch die geringe Substantivität des Dicaprylylethers auf dem Haar, was in der Regel zu unbefriedigenden Kämmkraftergebnissen führt.

Die Schrift WO 0234216 offenbart Zubereitungen, bestehend aus 40 bis 99Gew.-% Fettalkoholen und 1 bis 60 Gew.-% Wirkstoffen mit einer Wasserlöslichkeit bei 20°C von weniger als 1 g/l, mit der Maßgabe, dass sich die Mengenangaben zu 100Gew.-% ergänzen. Es handelt sich also um Zweistoffgemische.

Die Schrift EP 769934 offenbart Hautreinigungszubereitungen enthaltend alkylethoxylierte Sulfattenside mit einem EO-Grad von mindestens 2, bestimmte amphotere Tenside, ein Tensid auf der Basis von N-Acrylaminosäuren, bestimmte kationische Cellulosederivate, bestimmte Fettalkohole, wobei die Zubereitungen frei von anionischen Alkylsulfattensiden und polymeren Verdickern sind.

Es hat sich für den Fachmann nicht vorhersehbar herausgestellt, dass kosmetische und/oder dermatologische Körper- und Haarreinigungszubereitung enthaltend
a) Di-n-Octylether oder Di-n-Octylcarbonat -diese Stoffe sind beispielsweise unter dem Handelsnamen Cetiol LDO von Cognis (Di-n-Octylether) bzw. Cetiol CC von Cognis (Di-n-Octylcarbonat) erhältlich - und
b) mindestens zwei kationische Polymere, wobei es sich bei einem der Polymere um
   b1) ein quarternisiertes Polymer auf Basis von Cellulose, bevorzugt auf Basis von Hydroxypro-pylcellulose und besonders bevorzugt um Polyquaternium-10
   b2) ein quarternisiertes Guar Derivat, bevorzugt um Guar-Hydroxypropyltrimethylammoniumchlorid handelt, den Mängeln des Standes der Technik abhelfen. Solche Zubreitungen weisen gegenüber Zubereitungen des Standes der Technik eine deutlich bessere Pflegeleistung, die sich insbesondere in einer Verringerung der Kämmkraft und einem verbesserten Griffemfinden im nassen und trockenen Haar bniederschlägt.

Es wurde weiter gefunden, dass es bevorzugt ist, wenn zusätzlich ein oder mehrere waschaktive Tenside gewählt aus der Gruppe der anionischen, amphoteren oder nichtionischen Tenside enthalten sind. Weiter bevorzugt ist es, wenn das Tensid ein Aniontensid, besonders bevorzugt Ethersulfat, ganz besonders bevorzugt Laurylethersulfat darstellt. Dabei ist bevorzugt, wenn das Tensid in Konzentrationen von 4 bis 12Gew.% vorliegt, bezogen auf das Gesamtgewicht der Zubereitung.

Darüber hinaus ist es bevorzugt, wenn als zusätzliches Tensid ein amphoteres Tensid, besonders bevorzugt Alkylamidopropylbetain enthalten ist. Weiter bevorzugt ist es, wenn das quarternisiertes Polymer auf Basis von Cellulose in Konzentrationen von 0,01 bis 3%, besonders bevorzugt 0,1-2% vorliegt bezogen auf das Gesamtgewicht der Zubereitung. Bevorzugt ist es, wenn das Di-n-Octylcarbonat in Kombination mit Laurylalkohol enthalten ist. Diese Kombination ist unter dem Handelsnamen Cetiol LDO von Cognis erhältlich.

Bevorzugt ist es auch, wenn als quarternisiertes Polymer auf Basis von Cellulose Polyquaternium-10 in einer Konzentration von 0.01 bis 2 Gewichts-%, bevorzugt in einer Konzentration von 0.05 bis 1.5 Gewichts-% und besonders bevorzugt von 0.1 bis 1.0 Gewichts-%, jeweils bezogen auf das Gesamtgewicht der Zubereitung, enthalten ist. Unter Polyquaternium-10 werden erfindungsgemäß Polymere verstanden, welche gebildet werden aus quaternisierten Ammoniumsalz der Hydroxypropylcellulose, die mit einem Trimethylammonium substituierten Epoxid modifiziert ist. Es ist erfindungsgemäß vorteilhaft, wenn Polyquaternium-10 ein mittleres Molekulargewicht von 200000 bis 1000000 und eine Ladungsdichte von 0,8 bis 1,8 meq/g aufweist. Das erfindungsgemäß besonders bevorzugte Polyquaternium-10 ist das Ucare Polymer JR 400 der Firma Amerchol. Weiter bevorzugt ist es, wenn das quarternisierte Guar Derivat in einer Konzentration von 0.01 bis 3 Gewichts-%, bevorzugt in einer Konzentration von 0.05 bis 2 Gewichts-% und besonders bevorzugt in einer Konzentration von 0.01 bis 1 Gewichts-%, jeweils bezogen auf das Gesamtgewicht der Zubereitung, enthalten ist. Dabei ist es besonders bevorzugt, wenn als quarternisierte Guar Derivat eine oder mehrere Verbindungen gewählt aus der aus der Gruppe der Guar-Hydroxypropyltrimethylammoniumchloride, welche zur Klasse der Galactomannan 2-Hydroxypropyltrimethylammoniumchloridether gehören, ein mittleres Molekulargewicht von 600000 bis 2000000 aufweisen und eine Ladungsdichte von 0,4 bis 1,0 meq/g besitzen enthalten sind. Solche Guar-Hydroxypropyltrimethylammoniumchloride können aus der Jaguar-Serie der Firma Rhodia gewählt werden, wobei Jaguar Excel erfindungsgemäß ganz besonders bevorzugt ist.

Schließlich, aber nicht abschließend ist es bevorzugt, wenn zusätzlich eine Wachsdispersion mit einer durchschnittlichen Partikelgröße von 100nm - 30µm enthalten ist, besonders bevorzugt können Glycol Distearat oder PEG-3 Distearat als dispergierte Wachse verwendet werden.

Das Weglassen eines einzelnen Bestandteils beeinträchtigt die einzigartigen Eigenschaften der Gesamtzusammensetzung. Daher sind alle angegebenen Bestandteile der erfindungsgemäßen Zubereitungen zwangsläufig erforderlich, um die Erfindung auszuführen.

Erfindungsgemäße Zubereitungen können vorteilhaft zusätzlich Konservierungsstoffe, Farbstoffe und Pigmente, UV-Filter im Fall von Haarreinigungszubereitungen, Verdicker, auch polymere Verdicker, Lipide, Antioxidantien, Komplexbildner, weitere konditionierende Bestandteile, waschaktive Tenside enthalten.

Es ist bei all diesem im Einzelfalle möglich, daß die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so daß er weiß, daß bei solchen Über- oder Unterschreitungen der Boden der vorliegenden Erfindung nicht verlassen wird.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele

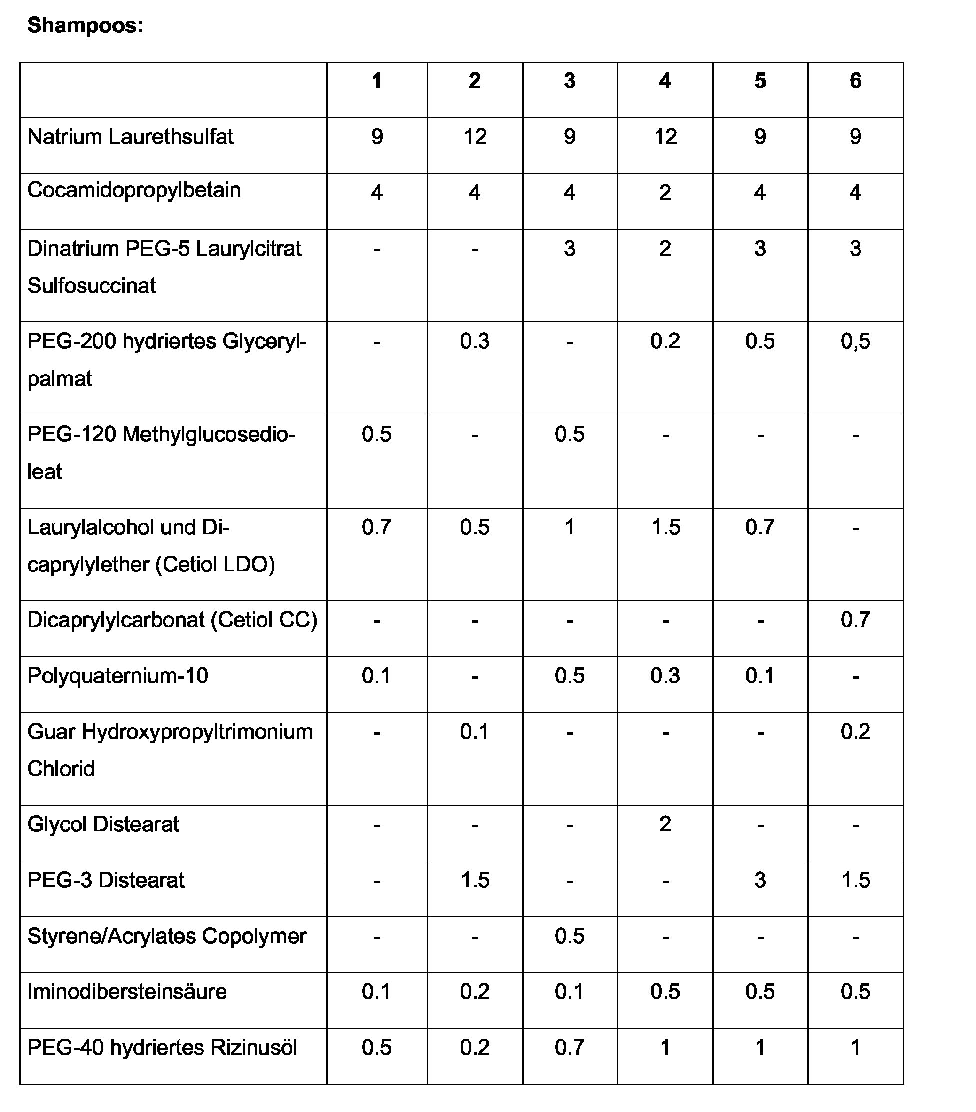

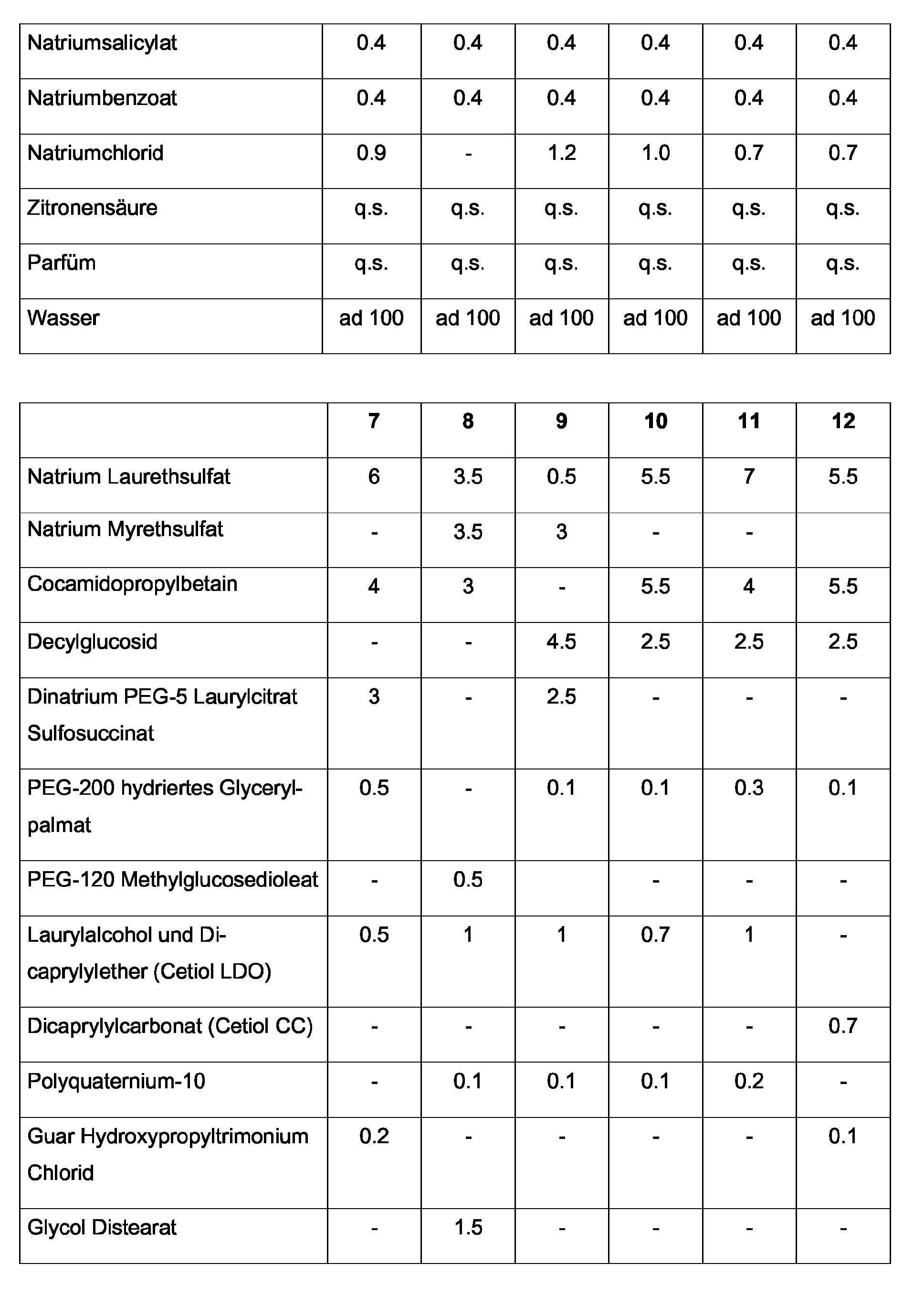

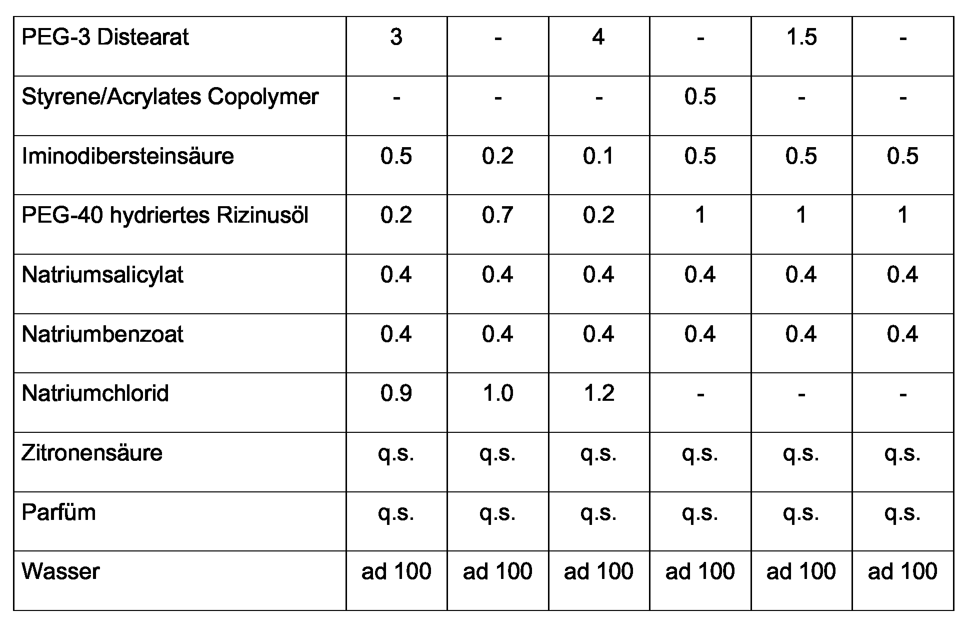

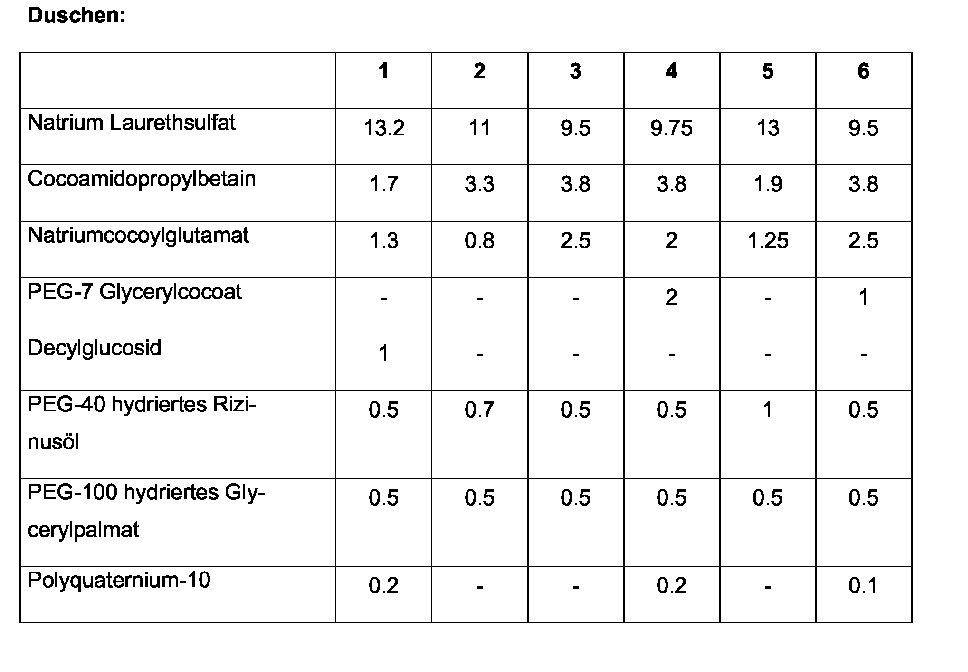

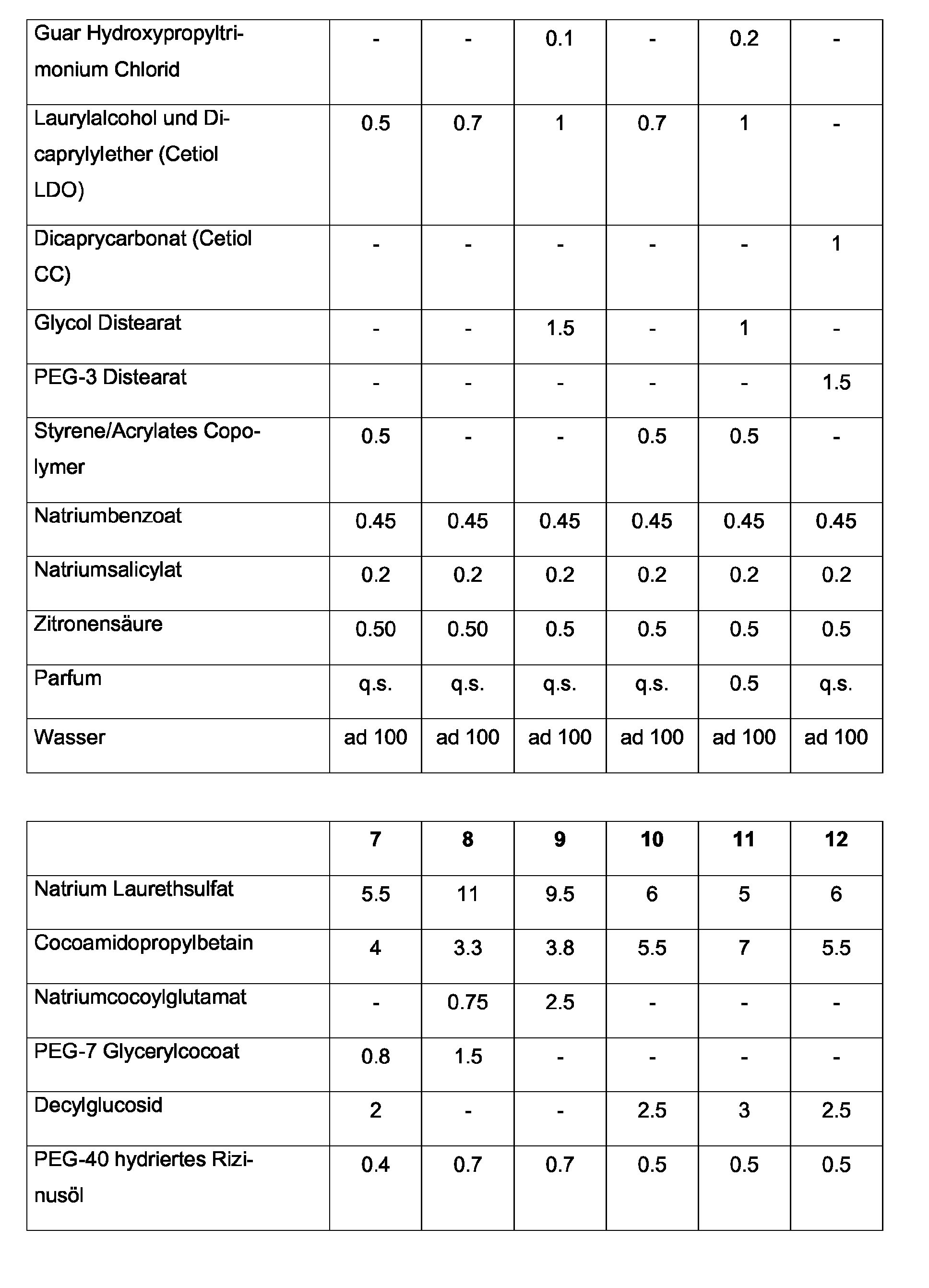

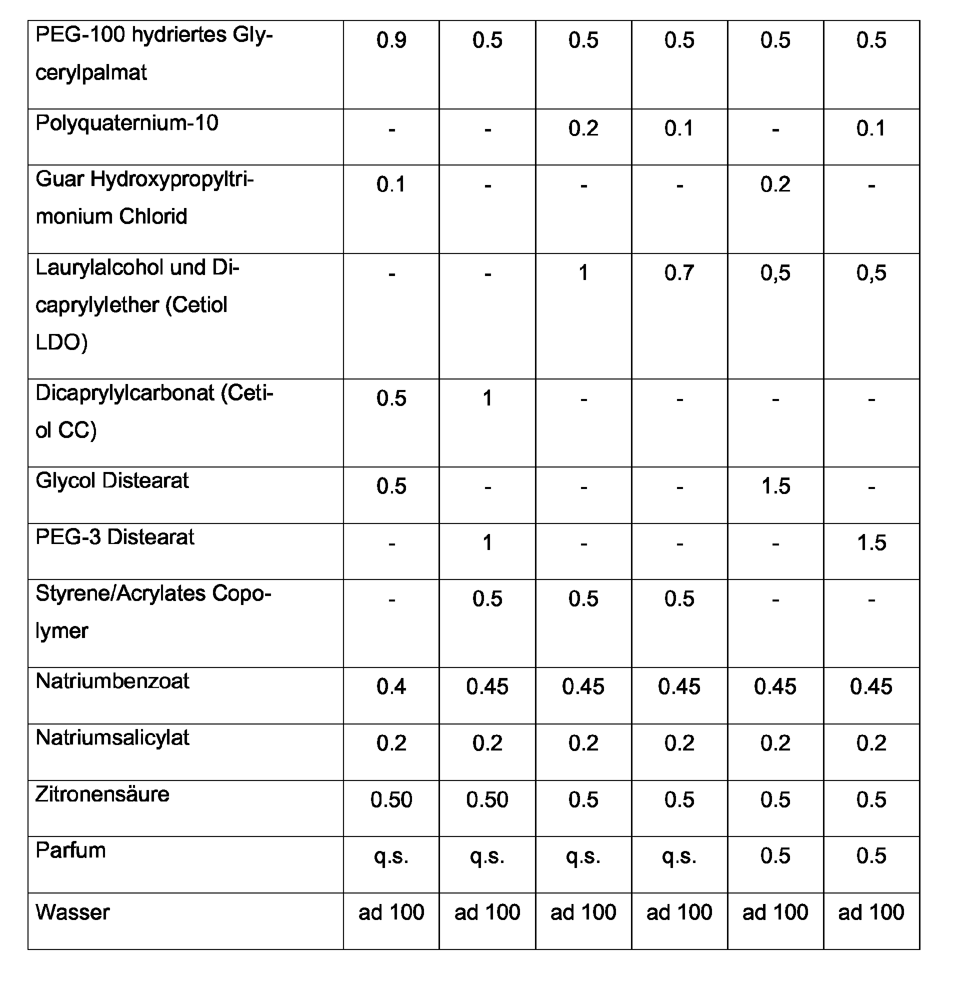

## Patentansprüche

1. Kosmetische und/oder dermatologische Körper- und Haarreinigungszubereitung enthaltend
a) Di-n-Octylether oder Di-n-Octylcarbonat
b) mindestens zwei kationische Polymere, wobei es sich bei einem der Polymere um
b1) ein quarternisiertes Polymer auf Basis von Cellulose, bevorzugt auf Basis von Hydroxypro-pylcellulose und besonders bevorzugt um Polyquaternium-10
b2) ein quarternisiertes Guar Derivat, bevorzugt um Guar-Hydroxypropyltrimethylammoniumchlorid handelt.

2. Zubereitung nach Anspruch 1 **dadurch gekennzeichnet, daß** zusätzlich ein oder mehrere waschaktive Tenside gewählt aus der Gruppe der anionischen, amphoteren oder nichtionischen Tenside enthalten sind.

3. Zubereitung nach einem der vorangehenden Ansprüche **dadurch gekennzeichnet, daß** das Tensid ein Aniontensid, besonders bevorzugt Ethersulfat, besonders bevorzugt Laurylethersulfat darstellt.

4. Zubereitung nach einem der vorangehenden Ansprüche **dadurch gekennzeichnet, daß** das Tensid in Konzentrationen von 4 bis 12Gew.% vorliegt.

5. Zubereitung nach einem der vorangehenden Ansprüche **dadurch gekennzeichnet, daß** als zusätzliches Tensid ein amphoteres Tensid, besonders bevorzugt Alkylamidopropylbetain enthalten ist.

6. Zubereitung nach einem der vorangehenden Ansprüche **dadurch gekennzeichnet, daß** das quarternisiertes Polymer auf Basis von Cellulose in Konzentrationen von 0,01 bis 3%, besonders bevorzugt 0,1-2% vorliegt

7. Zubereitung nach einem der vorangehenden Ansprüche **dadurch gekennzeichnet, daß** das Di-n-Octylcarbonat quaternisiertes in Kombination mit Laurylalkohol enthalten ist.

8. Zubereitung nach einem der vorangehenden Ansprüche **dadurch gekennzeichnet, daß** als quarternisiertes Polymer auf Basis von Cellulose Polyquaternium-10 in einer Konzentration von 0.01 bis 2 Gewichts-%, bevorzugt in einer Konzentration von 0.05 bis 1.5 Gewichts-% und besonders bevorzugt von 0.1 bis 1.0 Gewichts-%, jeweils bezogen auf das Gesamtgewicht der Zubereitung, enthalten ist.

9. Zubereitung nach einem der vorangehenden Ansprüche **dadurch gekennzeichnet, daß** das quarternisierte Guar Derivat in einer Konzentration von 0.01 bis 3 Gewichts-%, bevorzugt in einer Konzentration von 0.05 bis 2 Gewichts-% und besonders bevorzugt in einer Konzentration von 0.01 bis 1 Gewichts-%, jeweils bezogen auf das Gesamtgewicht der Zubereitung, enthalten ist.

10. Zubereitung nach einem der vorangehenden Ansprüche **dadurch gekennzeichnet, daß** als quarternisierte Guar Derivat eine oder mehrere Verbindungen gewählt aus der aus der Gruppe der Guar-Hydroxypropyltrimethylammoniumchloride, welche zur Klasse der Galactomannan 2-Hydroxypropyltrimethylammoniumchloridether gehören, ein mittleres Molekulargewicht von 600000 bis 2000000 aufweisen und eine Ladungsdichte von 0,4 bis 1,0 meq/g besitzen enthalten sind.

11. Zubereitung nach einem der vorangehenden Ansprüche **dadurch gekennzeichnet, daß** zusätzlich eine Wachsdispersion mit einer durchschnittlichen Partikelgröße von 100nm - 30µm enthalten ist.
